**Europäisches Patentamt**

**European Patent Office**

**Office européen des brevets**

(19)

(11) Veröffentlichungsnummer : **0 358 876 B1**

(12)

# EUROPÄISCHE PATENTSCHRIFT

(45) Veröffentlichungstag der Patentschrift :
**11.11.92 Patentblatt 92/46**

(51) Int. Cl.⁵ : **B65B 55/16,** A23L 3/00,
A61L 2/12, H05B 6/68,
H05B 6/78

(21) Anmeldenummer : **89112638.5**

(22) Anmeldetag : **11.07.89**

(54) **Arbeitsverfahren und Vorrichtung zum gleichmässigen Erwärmen mittels Mikrowelle.**

(30) Priorität : **10.09.88 DE 3830867**

(43) Veröffentlichungstag der Anmeldung :
**21.03.90 Patentblatt 90/12**

(45) Bekanntmachung des Hinweises auf die
Patenterteilung :
**11.11.92 Patentblatt 92/46**

(84) Benannte Vertragsstaaten :
**CH DE FR GB IT LI NL**

(56) Entgegenhaltungen :
**EP-A- 0 287 760**
**FR-A- 2 559 884**
**FR-A- 2 569 538**

(73) Patentinhaber : **HERMANN BERSTORFF**
**Maschinenbau GmbH**
**An der Breiten Wiese 3/5**
**W-3000 Hannover 61 (DE)**

(72) Erfinder : **Koch, Klaus, Dipl.-Phys.**
**Feldstrasse 2**
**W-3014 Laatzen 4 (DE)**

EP 0 358 876 B1

## Beschreibung

Die Erfindung betrifft ein Arbeitsverfahren mit den Merkmalen gemäß dem Oberbegriff von Patentanspruch 1.

Aus der DE-PS 34 47 544 ist eine Vorrichtung zum Pasteurisieren mittels Mikrowellen bekannt. Sie weist eine längliche Mikrowellenbehandlungskammer mit einem durch die Kammer geführten, endlosen Transportband auf. Die Behandlungskammer weist Mikrowelleneinspeisekanäle auf.

Diese Einrichtung benötigt exakt gleichmäßige nichtmetallische Transportbehälter für das Gut, die exakt auf dem Transportband positioniert werden müssen. Leichte Abweichungen von den vorgesehenen Positionen in Förderrichtung auf dem endlosen Transportband führen zu einer Verschiebung des gesamten Mikrowelleneinspeisevorganges, wodurch dann örtliche Verbrennungen nicht zu vermeiden sind.

Es ist die Aufgabe der Erfindung, ein Arbeitsverfahren vorzuschlagen, mit dem eine sehr gleichmäßige Erwärmung von in offenen Behältern gelagerten Produkten, z. B. chemische und pharmazeutische Produkte oder Fertiggerichte zwecks Durchführung eines Pasteurisiervorganges durchführbar ist. Insbesondere soll erreicht werden, daß die Anordnung der Behälter oder Packungen auf dem Transportband, die Packungslänge sowie die Abstände dazwischen frei gewählt werden können. Die Anlage soll an eine breite Produktpalette hinsichtlich der Behandlungsintensität und -dauer ohne großen Aufwand anpaßbar sein.

Die Aufgabe wird durch das im Kennzeichen von Anspruch 1 beschriebenen Arbeitsverfahrens gelöst. Eine Vorteilhafte Weiterbildung des Verfahren ist in Unteranspruch 2 angegeben. Eine für die Durchführung des Arbeitsverfahrens besonders geeignete Vorrichtung wird in Anspruch 3 beansprucht.

Durch die systematische Erfassung bzw. Registrierung des Anfanges und Endes der Behälter auf dem Transportband vor dem Einlaufen in die Anlage, wird eine dosierte Mikrowellenleistungseinspeisung in jeden Behälter durchführbar, ohne daß auf eine genaue Positionierung der Verpackungen untereinander und der dazwischen liegenden Abstände in Förderrichtung geachtet werden muß. Im Alltagsbetrieb einer derartigen Anlage ist dieser Vorteil von entscheidender Bedeutung.

Aber auch hinsichtlich einer Energieeinsparung bietet das erfindungsgemäße Verfahren wesentliche Vorteile. Die zum Erreichen einer vorbestimmten Produkttemperatur erforderliche Energie wird nur dann dem Produkt im Behälter bzw. dem verpackten Produkt zugeführt, wenn es sich unterhalb einer Öffnung der Mikrowelleneneinspeisekanäle befindet. Durch die Abschaltung der Mikrowelleneinspeisung, wenn kein Behälter unterhalb der Einspeiseöffnung ist, wird viel Energie eingespart.

Der größte Vorteil des Arbeitsverfahrens ist jedoch, daß gezielt hinsichtlich jeder einzelnen Packung eine Taktlänge bzw. eine Taktzeit eingehalten werden kann, um eine gleichmäßige Temperaturverteilung in den Packungen sicherstellen zu können.

Die Registrierung der Behälter, bevor diese in die Behandlungskammern einlaufen, hat wesentliche Vorteile. In der Behandlungskammer brauchen an den jeweiligen Einspeisekanälen keine an ein Regelgerät anzuschließende Positionsmelder angeordnet werden.

Die Anordnung derartiger Positionsmelder einschließlich ihrer Verkabelung in der, eine hohe Temperatur (z.B. 150° C) aufweisende, Behandlungskammer, ist sehr problematisch. Durch die hohe Temperatur in der Behandlungskammer haben Positionsmelder sowie deren Verkabelung nur eine geringe Lebensdauer. Störungen in der Energieeinspeisung und Brände in der Behandlungskammer sind die Folge. Zudem ist eine Sensorausbildung notwendig, die von den elektrischen Feldern der Mikrowellengeneratoren nicht ge- bzw. zerstört wird.

Die Wahl des An- und Abschaltpunktes bzw. die Inbezugsetzung des An- und Abschaltpunktes zur Behälterlänge und zur Öffnungsmitte der Einspeisekanäle erlaubt ein sehr individuelles Eingehen der Ein- und Ausschaltung der Mikrowelleneinspeisung. Auf diese Weise können verschiedeneste Packungslängen und Aufheizzeiten eingestellt werden. Auch die Intensivität der Mikrowellenbeaufschlagung (Höhe der Mikrowellenleistung) kann individuell bestimmt werden. Wird der Anschaltpunkt zu früh gewählt, d.h., bevor die Packung unter den Einspeisekanal läuft, entstehen örtliche Überhitzungen in den Packungsanfängen. Wenn der Anschaltpunkt zu spät gewählt wird, wird der Anfang der auf dem Transportband geführten Packung zu wenig erwärmt. Die Folge davon ist eine ungleichmäßige Erwärmung.

Die Leistungsauskoppelung kann gestoppt werden, bevor das Ende der Verpackung sich senkrecht unter der Austrittsöffnung des Einspeisekanals befindet, je nach der Länge und des Inhaltes der Verpackung.

Die Länge der zu behandelnden Behälter kann in den Rechnen eingegeben werden. In diesem Fall können jedoch nur Packungen gleicher Länge behandelt werden, aber ohne daß die Abstände dazwischen gleich sein müssen.

Eine Arbeitsweise wird aufgezeigt, die die Behandlung von unterschiedlich langen Behältern erlaubt, weil sowohl der Anfang als auch das Ende z.B. durch eine Abtasteinrichtung erfaßt und die Mikrowelleneinspeiseleistung entsprechend gesteuert wird.

2

Eine exakte Festlegung des An- und Abschaltpunktes wird offenbart, so daß unabhänging von der Behälterlänge eine gleichmäßige Aufheizung der Packungsanfänge und -enden einstellbar ist.

Aufgezeigt wird, von welchen Parametern die am Rechner voreingestellte, in das Produkt einzuleitende Mikrowellenleistung bzw. -energie abhängig gemacht werden kann.

In Anspruch 2 wird eine Weiterbildung des Anspruchs 1 aufgezeigt. Die Mikrowellenleistung, die zwischen dem An- und Abschaltpunkt in die Behälter oder Verpackungen eingespeist wird, kann taktweise eingespeist werden, während des Transportes des Behälters unterhalb der Austrittsöffnung der Einspeisekanäle. Die Einspeisetaktlänge kann beispielsweise zeitgleich mit der Abschaltpause der Mikrowelleneinspeiung gewählt werden. Die taktweise Mikrowelleneinspeisung wird bis zum Erreichen des Abschaltpunktes durchgeführt. Die taktweise Einspeisung hat den Vorteil einer besseren Temperaturvergleichmäßigung. Insbesondere wird jedoch damit erreicht, daß die Oberfläche des zu behandelnden Produktes keine Überhitzung und somit eine Schädigung erfährt.

Eine weitere Möglichkeit der Verbesserung der Temperaturvergleichmäßigung wird erreicht, wenn die Mikrowelleneinspeisung zwischen An- und Abschaltpunkt gezielt hinsichtlich der Leistungshöhe gesteuert wird, z.B. wenn mit 600 Watt gleich 50 % begonnen und in der Mitte des Behälters oder der Verpackung eine Erhöhung auf 100 %, d.h. auf 1,2 KW, stattfindet. Zum Ende der Verpackung wird dann die Leistung wieder abgesenkt auf 600 Watt.

Auch die Steuerung der Form der Leistung der eingespeisten Mikrowellen führt zu einer weiteren Vergleichmäßigung der Temperatur. Die Form der Leistung kann rechteckig, trapezförmig, dreieckförmig oder sinuskurvenförmig gewählt werden, jeweils in Abhängigkeit von der Oberflächenform des Produktes in dem Behälter bzw. in der Packung. Die Oberfläche des Produktes sollte in keinem Fall zu lange mit zu hoher Mikrowellenleistung beaufschlagt werden, weil z.B. eine Flüssigkeitsoberfläche (Fleisch mit Soße) bei einer zu hohen Energieeinleitung in Produkte mit schon höherer Temperatur (T = 70° C) in die Dampfphase läuft und dadurch die Verpackung zum Platzen bringen kann. Eine heterogene Oberfläche (z.B. Blumenkohl) ist eine größere Oberfläche und erlaubt daher eine bessere Energieverteilung in dem Produkt. Die Mikrowellenleistung gelangt dabei tiefer in das Produkt und kann sich somit besser verteilen (geringere Energiedichte).

Die Form der Leistung wird in vorteilhafter Weise so gestaltet, daß die Leistungsspitze mit zunehmender Produktoberflächentemperatur immer kürzer auf die Produktoberfläche einwirkt (Reihenfolge: rechteckförmig, trapezförmig, sinusförmig, dreieckförmig). Die Wahl der jeweiligen Leistungsform erfolgt daher in Abhängigkeit von der Temperaturentwicklung im Produkt, insbesondere jedoch hinsichtlich der Temperaturentwicklung der Oberfläche des Produktes.

Nachfolgend werden bevorzugte Ausführungsbeispiele der Erfindung anhand der Zeichnungen erläutert. Es zeigen:

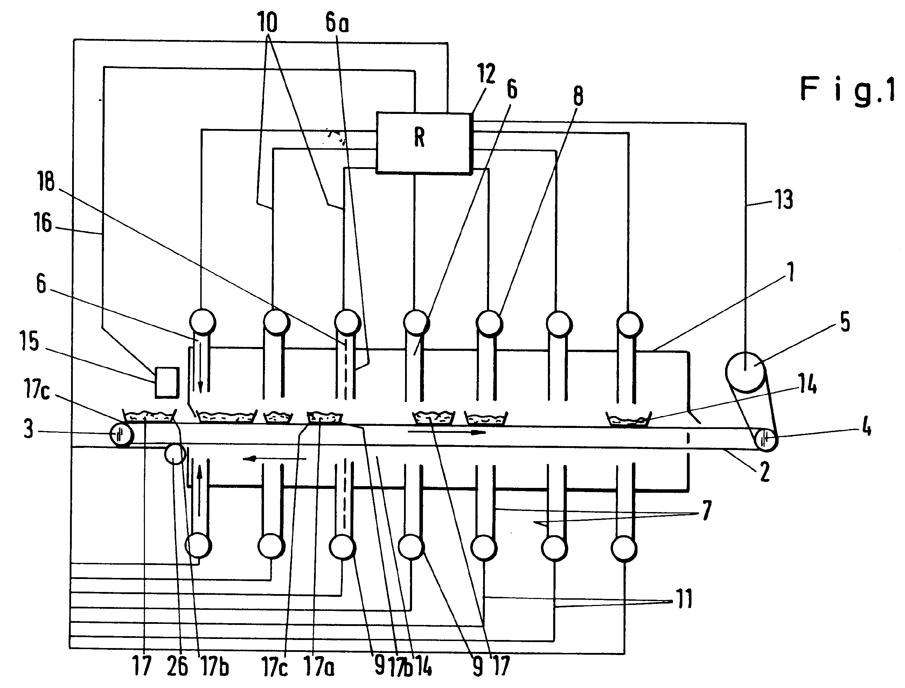

Fig. 1 einen schematisierten Längsschnitt durch eine Mikrowellenanlage.

Fig. 2 zeigt schematisiert den Verlauf der Energieeinspeisung.

Fig. 3 eine Darstellung der Temperaturverteilung in einer Packung.

Fig. 4 eine Darstellung der Temperaturverteilung gemäß dem Stand der Technik mit einer Mikrowelleneinspeisung ohne Steuerung der einzelnen Generatoren.

Fig. 5 eine Darstellung der Temperaturverteilung mit einer Taktung während des Mikrowelleneinspeisevorganges der einzelnen Generatoren.

Fig. 6 eine Darstellung der unterschiedlichen Mikrowellenleistungsformen und -höhen, auch in Verbindung mit einer Taktung während des Einspeisevorganges der einzelnen Generatoren.

Durch die Behandlungskammer 1 wird ein endloses Transportband 2 geführt und um die Rollen 3 und 4 umgelenkt. An der Umlenkrolle 4 ist ein Antrieb 5 angeschlossen.

In die Behandlungskammer münden obere Mikrowelleneinspeisekanäle 6 und untere -kanäle 7 ein, an die jeweils obere Generatoren 8 bzw. untere Generatoren 9 für die Erzeugung der Mikrowellenenergie angeschlossen sind.

Die oberen Generatoren 8 sind mittels der Leitungen 10 und die unteren Generatoren 9 mittels der Leitungen 11 an einen Rechner 12 angeschlossen.

Der Antrieb 5 ist durch eine Leitung 13 mit dem Rechner verbunden.

Zwecks Registrierung der exakten Positionen der Behälter 17 auf dem Transportband 2 wird eine Reflexlichtschranke 15 vorgesehen, die mittels der Leitung 16 mit dem Rechner 12 und mit einem allgemein bekannten Weggeber 26 verbunden ist.

Nachfolgend wird die Funktionsweise der Mikrowellenanlage erläutert:

Die Position der Packung 17a auf dem Transportband 3, d.h. insbesondere der Anfang 17b und das Ende 17c wird durch die Registriereinrichtung 15 erfaßt und an den Rechner 12 gegeben.

Die Registriereinrichtung ist in diesem Fall eine Reflexlichtschranke 15, die in Verbindung mit einem an den Rechner 12 angeschlossenen Weggeber 26 arbeitet. Der Weggeber 26 wandelt die Bandbewegung in elek-

trische Impulse um, die so dimensioniert werden können, daß z.B. ein Impuls pro Millimeter Bandbewegung an den Rechner gegeben wird. Mit dieser Auflösung können die Schaltpunkte zum An- und Abschalten der Mikrowellenleistung bezüglich der Behälterlänge und der Öffnungsmitte 18 millimetergenau definiert werden.

Bei der exakten Registrierung des Anfanges 17b und des Endes 17c errechnet der Rechner 12 die An- und Abschaltpunkte der Mikrowellenleistung im Verhältnis zur Behälterposition auf dem Band.

Wenn die Packung 17a mit ihrem Anschaltpunkt 23 unter der Öffnungsmitte des Einspeisekanals 18 hindurchläuft, schaltet der Rechner 12 die Mikrowellenleistung an. Die Einschaltung der Mikrowelleneinspeisung erfolgt aufgrund der Berechnung des von der Packung 17 mit dem Transportband 3 zurückgelegten Weges bis unterhalb der Öffnungsmitte 18 des Einspeisekanals 6a.

Nach Passieren des Ausschaltpunktes unterhalb der Öffnungsmitte 18 wird die Mikrowellenauskoppelung aus Kanal 6a durch den Rechner 12 bei Erreichen des Ausschaltpunktes 24 vor dem Behälterende ausgeschaltet.

Dieser Vorgang wird durchgeführt, ohne daß eine Abtastung des Packungsanfanges 17b oder -endes 17c in der Kammer 1 erfolgen muß. Lediglich durch die Registrierung der Position der Pakkung 17 auf dem Transportband 3 vor dem Eintreten in die Behandlungskammer ermittelt der Rechner jeweils den exakten Zeitpunkt des Beginns und der Beendigung der Mikrowellenauskoppelung für jeden einzelnen Kanal 6, 7 und für jede einzelne Packung. Die nicht veränderbare Anordnung der Einspeisekanäle 6 und deren Abstände 25 zueinander wird bei dem Durchlauf der Packungen 17 durch die Behandlungskammer 1 ebenfalls von dem Rechner 12 berücksichtigt.

Jeder an einen Einspeisekanal 6 bzw. 7 angeschlossene Generator 8 und 9 wird hinsichtlich seiner Leistung sowie seiner Einschaltzeit von dem Rechner 12 gesteuert. Die Steuerung erfolgt aufgrund der Registrierung durch die Reflexlichtschranke 15 unabhängig von der Geschwindigkeit des Transportbandes 3 bzw. entsprechend der Eingabe der Werte für die Packungslänge, dem Packungsinhalt, dem Gewicht und der Dichte des zu behandelnden Produktes, den dielektrischen Werten des Produktes und der geforderten Temperaturerhöhung delta-T.

Hervorzuheben ist, daß die Mikrowelleneinkoppelung der Generatoren 8 und 9 für jeden einzelnen Einspeisekanal 6, 7 und für jede einzelne Packung aufgrund obiger Parameter vom Rechner 12 gesteuert wird. Es spielt daher keine Rolle, ob die Packungen in einem exakten Abstand zueinander auf das Transportband 3 liegen. Jede Packung wird individuell registriert und auch jeweils die Mikrowelleneinspeisetaktlänge der Generatoren für jede Packung gesteuert. Es ist dabei nicht von Bedeutung, ob eine lange oder kurze Packung mit kleinem oder großem Abstand zueinander im Wechsel auf das Transportband gelegt wird.

Ein positionierter Behälterinhalt kann z.B. in Förderrichtung aufgeteilt in drei verschiedene Lebensmittelprodukte, wie Reis + Fleisch mit Soße + Gemüse, durch eine den Produktgrenzen zugeordnete Vorgabe des Ein- und Ausschaltpunktes der Mikrowelleneinspeisung ebenfalls gleichmäßig erwärmt werden, obwohl unterschiedliche Dichten, Gewichte und dielektrische Werte der einzelnen Komponenten vorliegen.

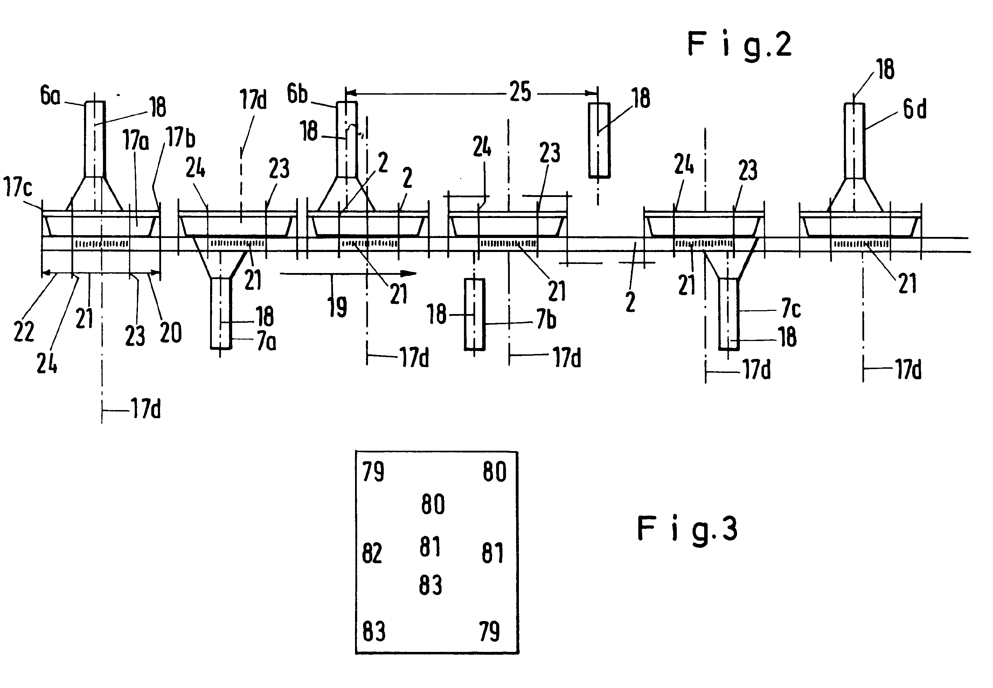

In Fig. 2 wird in schematisierter Weise ein bevorzugtes Beispiel einer Arbeitsweise aufgezeigt, welches zu noch wirtschaftlicherem Arbeiten und zu einer äußerst gleichmäßigen Aufheizung der Packungen und der Packungsränder führt.

Der Behälter 17a auf dem Transportband 2 wird in Richtung des Pfeiles 19 durch die hier nicht gezeigte Behandlungskammer geführt.

Zunächst läuft der Behälter 17a unter den Einspeisekanal 6a hindurch. Der Anschaltpunkt 23 der Mikrowelleneinspeiseleistung wird in Bezug zur Behälterlänge und zur axialen Öffnungsmitte 18 des Einspeisekanals 6a gesetzt und ist abhängig vom zu pasteurisierenden Produkt im Behälter.

Die Wegstrecke, die der Behälter zurückgelegt hat, bis die Energieeinspeisung eingeschaltet wird, ist mit 20 gekennzeichnet. Die Wegstrecke, die gleichzusetzen ist mit der Anschaltzeit bzw. der Taktzeit der Mikrowelleneinspeisung in Abhängigkeit von der Bandgeschwindigkeit, wird mit 21 gekennzeichnet. Die Strecke 22 steht für den Weg, den der Behälter auf dem Transportband 2 zurücklegt, um aus dem Bereich der Einspeisekanäle 6 und 7 zu gelangen.

Der Abschaltpunkt der Mikrowelleneinspeisung wird mit 24 bezeichnet. Der Einschaltvorgang wird in diesem Fall mit Passieren des Einschaltpunktes unter/über der Öffnungsmitte 18 der Einspeisekanäle eingeleitet. Dieser Zustand wird dargestellt bei dem unteren Einspeisekanal 7b. Der Ausschaltpunkt 24 ist hinter der Öffnungsmitte 18 des unteren Einspeisekanal 7b. Je nach dem zu behandelnden Produkt kann der Einschaltpunkt 23 und der Ausschaltpunkt 24, d.h. der Anschaltweg (Taktlänge) unterschiedlich gewählt werden.

Wenn eine geringere Mikrowellenbeaufschlagung zweckmäßig ist, werden die Wegstrecken 20 und 22 verlängert, wodurch die Taktzeit 21, d.h. die Beaufschlagungsdauer verkürzt wird. Die Taktlänge 21 wird entsprechend der Länge der Behälter 17 gewählt. Die jeweilige Länge der Wegstrecken 20 bzw. 22 ist für eine bessere Temperaturverteilung in den Behältern wichtig.

Es kann bei einem Behälterinhalt, z.B. ein Fertiggericht mit einem relativ großen Soßenanteil (Wasseranteil), zweckmäßig sein, den Einschaltpunkt 23 auf den Behälteranfang 17c und den Ausschaltpunkt 24 mit dem Behälterende 17b zusammen zu legen.

Der An- und Abschaltpunkt 23, 24 wird in jedem Einzelfall, d.h. je nach dem zu behandelndem Produkt und der Behälterlänge, gewählt, jedoch stets bezogen auf die Behälterlänge und die Öffnungsmitte 18 der Einspeisekanäle.

Die Steuerung der Mikrowelleneinspeisung erfolgt, wie eingangs geschildert, aufgrund der Registrierung der Position des Behälters 17 auf dem Transportband 2, und zwar vor dem Einlaufen in die Behandlungskammer 1. Aufgrund einer entsprechenden Programmierung des Rechners 12, unter Berücksichtigung der Geschwindigkeit des Transportbandes 1, der Behälterlänge, der gewünschten Taktzeit 21 und der Energiemenge, wird die Leistungsauskoppelung jedes einzelnen Einspeisekanals 6 und 7 gesteuert. Der Rechner 12 kann dabei auch aufgeteilt werden in einzelne, den Magnetrons zugeordnete Mikroprozessoren und einem Führungsrechner.

Aufgrund dieses Verfahrens und der für die Durchführung des Verfahrens eingesetzten Einrichtung wird erstmalig

- eine gleichmäßige Erwärmung eines homogen gefüllten Behälterinhaltes (z.B. Nudeln mit Soße) gewährleistet, weil die Taktzeit 21 wählbar ist,
- eine gleichmäßige Erwärmung eines mit z.B. drei unterschiedlichen Produkten (Reis, Fleisch mit Soße, Gemüse) befüllten Behälter gewährleistet,
- erreicht, daß unterschiedlich lange Behälter mit beliebigen Abständen auf das Transportband gesetzt werden können,
- sichergestellt, daß die Anlage eine höchstmögliche Ausnutzung der teuren Mikrowellenenergie erlaubt, weil aufgrund der Rechnersteuerung die Taktzeit, die Behälterlänge und die Bandgeschwindigkeit gezielt berücksichtigt werden und jeder Einspeisekanal separat gesteuert wird.

Wenn die zwischen dem An- und Abschaltpunkt eingespeisten Mikrowellen taktweise eingegeben werden, wird eine weitere Verbesserung der Temperaturvergleichmäßigung erreicht.

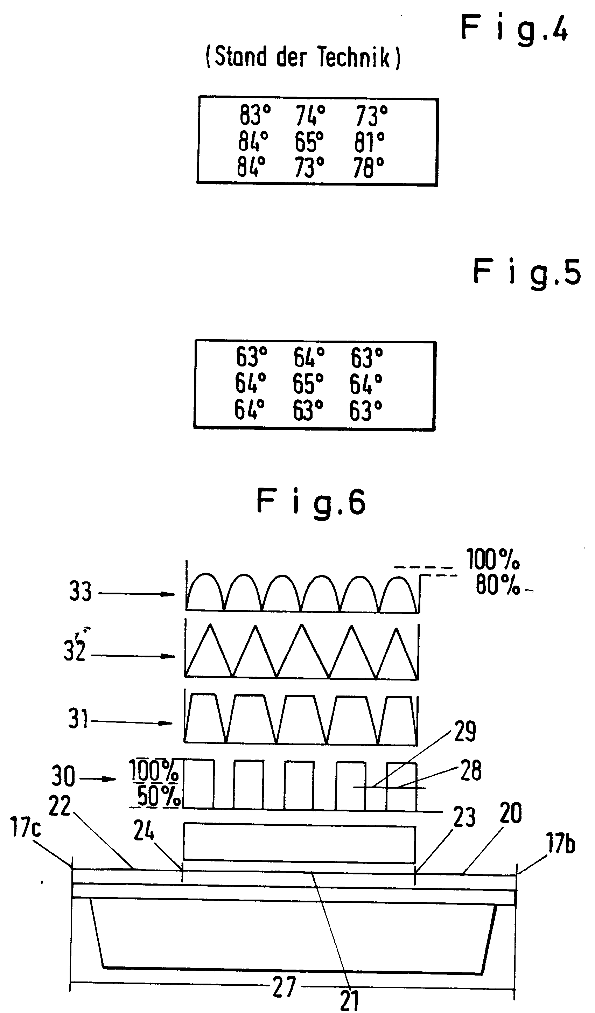

Bei einer Taktlänge von z.B. 4 s und einer Pause von ebenfalls 4 s wurde erreicht, daß das bei herkömmlicher Dauerleistung gemäß dem Stand der Technik im Vergleich zu den Randzonen um ca. 20 °C tiefer liegende Produktzentrum (Fig. 4) auf gleiche Temperatur mit den Randzonen angehoben werden konnte (Fig. 5).

Entsprechend den Ergebnissen, gezeigt in Fig. 4 und 5, ist es durch Anwendung des beschriebenen Arbeitsverfahrens (gepulste Mikrowellenleistung) auch bei langsamen Fördergeschwindigkeiten möglich, ein homogenes Temperaturfeld im Behandlungsgut zu erreichen.

Ein weiterer positiver Effekt hat sich dahingehend herausgestellt, daß bei versiegelten Kunststoffpackungen die Aufblähung der Siegelfolie durch inneren Überdruck vernachlässigbar klein wird. Während des Leistungstaktes beginnt die Kopfraumausdehnung nach einigen Sekunden, um nach etwa 5 s kritisch groß zu werden. Durch die etwa ebenso große Einspeisungspause, verschwindet diese Aufblähung wieder, um bei erneutem Leistungsimpuls wieder von 0 an zu beginnen.

Zusammen mit einem zur Packungslänge definiertem Ein- und Ausschalten dieses Impulsbetriebes wird somit eine gleichmäßige Erwärmung des Produktes gewährleistet.

Außerdem wird durch die Taktung der Einergie zwischen den An- und Abschaltpunkten 23 und 24, im Gegensatz zu einem Dauerbetrieb gemäß dem Stand der Technik, die Eindringgeschwindigkeit der Mikrowellenleistung in das Produkt erheblich erhöht, so daß die Produktoberfläche weniger überhitzt wird.

In Fig. 6 wird die Form der Mikrowellenleistung, die zwischen dem Anschaltpunkt 24 und dem Abschaltpunkt 23 durch die Einspeisekanäle 6, 7 in den Behälter 17 eingegeben wird, bildlich dargestellt.

Die Form der Leistung beeinflußt die Temperatur der Produktoberfläche. Die Leistungsspitze sollte mit zunehmender Produktoberflächentemperatur kürzer auf die Produktoberfläche einwirken.

Die rechteckförmige Leistungsform 30 bewirkt die stärkste Beeinflussung der Produktoberfläche. Die trapezförmige Leistungsform 31 vermindert diese Beeinflussung und die dreieckförmige Leistungsform 32 führt eine weitere Verringerung der Oberflächeneinwirkung auf das Produkt herbei.

Wenn zusätzlich zu der Wahl einer sinuskurvenförmigen Leistungsform 33 eine Verminderung der Leistungshöhe von 100 % auf 80 % vorgenommen wird, werden sehr gute Ergebnisse erzielt.

Durch einen Impulsbetrieb der Anlage, d.h. ein impulsartiges Eingeben der Mikrowellenenergie (zwischen den An- und Abschaltpunkten 23, 24), wurde in überraschender Weise festgestellt, daß versiegelte Packungen bis zu Produkttemperaturen von 85 - 95° C nicht mehr aufplatzten, was einen erheblichen technischen Fortschritt darstellt.

EP 0 358 876 B1

Beispiel:

In die Lebensmittelverpackung aus z.B. einer tiefgezogenen und versiegelten Kunststoffolie wird ein vorgefertigtes Lasagne-Gericht eingegeben, das einem Pasteurisiervorgang unterworfen werden soll, um Keime abzutöten zwecks Verlängerung der Verbrauchszeit bzw. der Haltbarkeit.

Die Packungen haben folgende Abmessungen:

Länge: 190 mm
Breite: 140 mm
Höhe: 28 mm
Gewicht: 400 g

Nachfolgend wird ein Beispiel beschrieben, welches in Übereinstimmung mit der Lehre des ersten Patentanspruches durchgeführt wurde.

Um die für einen Pasteurisiervorgang erwünschte Produkttemperatur von 80° C zu erhalten, wird ein Energiebedarf von 0,025 kWh pro 400 g Packung für die Durchlaufzeit errechnet, wobei von gleichen Packungsdimensionen und von einer Produkteingangstemperatur von 40° C ausgegangen wird.

Der Rechner 12 wird folgendermaßen programmiert:

a) alle oberen Generatoren 8 und unteren Generatoren 9 werden auf eine Leistungsabgabe von je 1,2 kW programmiert, so daß von unten und von oben jeweils durch die Einspeisekanäle 6 und 7 zusammen eine Mikrowellenleistung von 1,2 kW auf jede Lasagne-Packung während der Taktzeit 21 einwirkt.

b) Es sind sieben obere und sieben untere Einspeisekanäle 6 und 7 vorhanden. Die Gesamtbeaufschlagungszeit von einer Minute wird aufgeteilt auf 8,5 Sekunden pro oberer und unterer Einspeiseöffnung, d.h. die Taktzeit 21 der Lasagne-Packung vom Einschaltpunkt 23 bis zum Ausschaltpunkt 24 beträgt 8,5 Sekunden.

Es wird somit eine Gesamtenergiebeaufschlagungszeit von 7 x 8,5 Sekunden = ca. 1 Minute bei konstanter Mikrowellenleistungsabgabe von 1,2 kW verwirklicht, und zwar jeweils mit der vollen erforderlichen Leistung von 1,2 KW pro Magnetron und pro Packung.

Der Abstand von der Austrittsöffnung der Einspeisekanäle 6 und 7 zur Produktoberfläche beträgt 50 mm.

Zur Anwendung gelangte eine Mikrowellenanlage, die mit einer Wellenlänge von 12 cm bzw. 120 mm arbeitet (Frequenz von 2.450 GHz).

Eine typische Temperaturverteilung in der Verpackung wird in Fig. 3 wiedergegeben.

Aus Fig. 3 wird ersichtlich, daß die Temperaturdifferenz in der Packung, insbesondere hinsichtlich der Randbereiche zur Mitte nur gering ist, so daß ein Verbrennen der Randbereiche vermieden wird. Es wurde eine sehr gleichmäßige Keimabtötung über die gesamte Fläche festgestellt.

**Bezugszeichenliste**

1 = Behandlungskammer
2 = Transportband
3 = Umlenkrollen
4 = Umlenkrollen
5 = Antrieb
6 = obere Einspeisekanäle
7 = untere Einspeisekanäle
8 = Generator, obere
9 = Generator, untere
10 = Leitung, oben
11 = Leitung, unten
12 = Rechner
13 = Leitung, Antrieb
14 = Markierungsleisten auf Transportband
15 = Registriereinrichtung (Reflexlichtschranke)
16 = Leitung
17 = Lebensmittelbehälter
17a = Behälter
17b = Behälteranfang
17c = Behälterende
17d = Behältermitte
18 = Öffnungsmitte (axialer Mittelpunkt des Einspeisekanals)

19 = Pfeil
20 = Weg
21 = Taktzeit
22 = Weg
23 = Anschaltpunkt
24 = Abschaltpunkt
25 = Einspeisekanalabstand
26 = Weggeber
27 = Packungslänge
28 = Impulslänge
29 = Pausenlänge
30 = rechteckförmige Leistungsform
31 = trapezförmige Leistungsform
32 = dreieckförmige Leistungsform
33 = sinuskurvenförmige Leistungsform

**Patentansprüche**

1. Verfahren zum gleichmäßigen Erwärmen von Produkten mittels Mikrowellen, wobei die zu behandelnden Produkte, wie z.B. chemische oder pharmazeutische Produkte oder Fertiggerichte, in offenen oder abgeschlossenen, Mikrowellen durchlässigen Behältern auf einem kontinuierlich fördernden, endlosen Transportband durch eine Behandlungskammer geführt werden, in der senkrecht zum Transportband ausgerichtete Einspeisekanäle von Mikrowellen6-Generatoren angeordnet sind, deren Energieabgaben durch einen Rechner steuerbar sind,
**dadurch gekennzeichnet,**
daß der Anfang sowie das Ende eines auf dem Transportband geführten Behälters vor, bzw. beim Einlauf in die Behandlungskammer registriert und an den Rechner weitergeleitet wird,
daß die Mikrowellengeneratoren nur dann eingeschaltet werden, wenn sich Behälter unter den Öffnungen der Einspeisekanäle befinden, und
daß der genaue Anschalt- und Abschaltpunkt der einzelnen Mikrowellen-Generatoren sowie deren Energieabgaben von dem Rechner in Abhängigkeit von der in dem Behälter befindlichen Produktmenge, von der Beschaffenheit des Produktes, wie z.B. dielektrische Werte, Dichte, von der geforderten Temperaturerhöhung, von der Bandgeschwindigkeit des Transportbandes und von der Behälterlänge ermittelt und geregelt werden.

2. Verfahren nach Anspruch 1
**dadurch gekennzeichnet,**
daß die zwischen dem Anschaltpunkt (24) und dem Abschaltpunkt (23) von den einzelnen Generatoren (8, 9) durch die Einspeisekanäle (6, 7) abgegebene Mikrowellenleistung taktweise, in beliebiger Höhe und/oder in beliebiger Form (30, 31, 32, 33) in die Behälter (17) oder Packungen eingespeist wird.

3. Vorrichtung zur Durchführung des Verfahrens nach den Patentansprüchen 1 und 2 bestehend aus einer länglichen Behandlungskammer, durch die ein endloses Transportband läuft und mit durch die Wandung der Kammer von oben ragenden Einspeisekanälen, an die Mikrowellengeneratoren angeschlossen sind,
**dadurch gekennzeichnet,**
daß eine außerhalb der Behandlungskammer (1) angeordnete, den Anfang (17b) und das Ende (17c) eines Behälters (17) oder einer Verpackung auf dem laufenden Transportband (2) vor dem Einlaufen registrierende, an einen Rechner angeschlossene Einrichtung (15) vorgesehen wird,
daß ein die Geschwindigkeit des Transportbandes erfassender Weggeber (26) vorgesehen wird, der ebenfalls an den Rechner (12) angeschlossen ist,
daß jeweils an jedem Einspeisekanal (6, 7) ein hinsichtlich seiner Leistungsabgabe individuell regelbarer Mikrowellengenerator (8, 9) angeordnet ist, und
daß die Mikrowellengeneratoren (8, 9) an den Rechner angeschlossen sind, der diese an- und abschaltet sowie ihre Leistungsabgabe regelt.

4. Vorrichtung nach Anspruch 3
**dadurch gekennzeichnet,**

7

daß die den Anfang (17b) und das Ende (17c) der Behälter (17) oder der Verpackungen vor oder beim Einlaufen in die Behandlungskammer (1) registrierende Einrichtung (15) als eine, mit dem Weggeber (26) zusammenwirkende, die Position der Behälter (17) oder Verpackungen auf dem Transportband (2) für den An- und Abschaltvorgang der einzelnen Generatoren (8, 9) an den Rechner (12) gebende, Reflexionslichtschranke ausgebildet ist.

5. Vorrichtung nach Anspruch 3
   **dadurch gekennzeichnet,**
   daß die den Anfang (17b) und das Ende (17c) der Behälter (17) oder Verpackungen auf dem laufenden Transportband (2) registrierende an den Rechner (12) angeschlossene Einrichtung (15) als mechanische Taster, optische Taster, als auf Laserstrahlen basierender Taster, als Staudruckluftdüse, als Näherungsschalter oder als Ultraschall- bzw. Infrarottaster ausgebildet ist.

6. Vorrichtung nach Anspruch 3
   **dadurch gekennzeichnet,**
   daß jeweils ein senkrecht von oben in die Behandlungskammer (1) hineinragender Einspeisekanal (6) im Wechsel mit einem senkrecht von unten in die Kammer (1) ragenden Einspeisekanal (7) in Arbeitsrichtung der Anlage (1) hintereinander, oder
   daß jeweils einem senkrechten oberen Einspeisekanal (6) ein senkrechter unterer Einspeisekanal (7) gegenüber angeordnet ist.

## Claims

1. Method for evenly heating up products by means of microwaves, with the products to be treated, e.g. chemical or pharmaceutical products or ready-made meals, being transported in open or closed microwave-permeable containers on a continuously conveying, endless conveyor belt through a treatment chamber, in which supply channels of microwave generators the energy output of which is controllable by a computer are arranged vertically to the conveyor belt,
   **characterized in that**
   both the beginning and the end of the container that is transported on the conveyor belt are registered before and upon entering the treatment chamber and passed on to the computer,
   that the microwave generators are switched on only when the containers are below the openings of the supply channels, and
   that the precise switch-on and switch-off points of the individual microwave generators as well as their energy output are calculated and controlled by the computer in dependence on the amount of product in the container, the properties of the product, such as dielectric values, density, the required temperature increase, the belt speed of the conveyor belt, and the container length.

2. Method as per claim 1
   **characterized in that**
   the microwave energy is supplied intermittently and of any quantity and/or form (30, 31, 32, 33) to the containers (17) or packages by the individual generators (8, 9) via the supply channels (6, 7) between the switch-on point (23) and the switch-off point (24).

3. Device for carrying out the method as per patent claims 1 and 2, consisting of an oblong treatment chamber through which a continuous conveyor belt is led and which is provided with supply channels that reach through the chamber wall from above and have microwave generators connected to it,
   **characterized in that**
   a device (15) which is arranged outside the treatment chamber (1), which registers the beginning (17b) and the end (17c) of a container (17) or a package on the running conveyor belt (2) before the container enters the treatment chamber, and which is connected to a computer is provided,
   that a position transmitter (26) that records the conveyor belt speed and is also connected to the computer (12) is provided,
   that a microwave generator (8, 9) the energy output of which is individually controllable is arranged at each supply channel (6, 7), and that the microwave generators (8, 9) are connected to the computer which switches them on and off and controls their energy supply.

4. Device as per claim 3
**characterized in that**
the device (15) that registers the beginning (17b) and the end (17c) of the containers (17) or the packages before and at their entry into the treatment chamber (1) is designed as a reflection light barrier that acts together with the position transmitter (26) and passes on the position values of the containers (17) or packages on the conveyor belt (2) for the switch-on and switch-off processes of the individual generators (8, 9) to the computer (12).

5. Device as per claim 3
**characterized in that**
the device (15), which is connected to the computer (12) and serves for registering the beginning (17b) and the end (17c) of the containers (17) or packages on the running conveyor belt (2) is designed as mechanical pushbutton, optical switch, laser-based switch, ram air jet, proximity switch, ultrasonic or infrared switch.

6. Device as per claim 3
**characterized in that**
supply channels (6) reaching vertically into the treatment chamber (1) from above are arranged alternately with supply channels (7) reaching vertically into the chamber from below one behind the other in working direction of the line (1), or that one vertical top supply channel (6) and one vertical bottom supply channel (7) are always arranged one opposite the other.

**Revendications**

1. Procédé pour le chauffage uniforme de produits au moyen de micro-ondes, les produits à traiter, comme par exemple des produits chimiques ou pharmaceutiques ou des plats cuisinés contenus dans des récipients ouverts ou fermés mais perméables aux micro-ondes, étant menés sur une bande transporteuse sans fin circulant en continu à travers une chambre de traitement comprenant des canaux d'alimentation alignés verticalement par rapport à la bande transporteuse et connectés à des générateurs micro-ondes, dont les quantités d'énergie émises peuvent être commandées par ordinateur,
**caractérisé**
en ce que le début ainsi que la fin d'un récipient placé sur la bande transporteuse avant ou bien lors de son entrée dans la chambre de traitement sont repérés et transmis à l'ordinateur,
en ce que les générateurs micro-ondes ne sont mis en marche que lorsque des récipients se trouvent au-dessous des ouvertures des canaux d'alimentation et en ce que les points précis de branchement et de coupure des générateurs micro-ondes individuels ainsi que leurs quantités d'énergie émises sont déterminés et réglés par l'ordinateur en fonction de la quantité de produit se trouvant dans le récipient, des propriétés du produit, comme par ex. les valeurs diélectriques et la densité, de l'augmentation requise de la température, de la vitesse de la bande transporteuse et de la longueur du récipient.

2. Procéde selon revendication 1
**caractérisé**
en ce que la puissance micro-ondes introduite dans les récipients (17) ou emballages entre le point de branchement (23) et celui de coupure (24) par les générateurs individuels (8, 9) à travers les canaux d'alimentation (6, 7) peut être alimentée de façon cadencée, dans une grandeur quelconque et/ou dans une forme quelconque (30, 31, 32, 33).

3. Appareil pour la mise en oeuvre du procédé selon les revendications 1 et 2 comprenant une chambre de traitement oblongue à travers laquelle passe une bande transporteuse sans fin et des canaux d'alimentation qui pénétrent par le haut la paroi de la chambre et qui sont connéctes aux générateurs micro-ondes,
**caractérisé**
en ce qu'un dispositif (15), arrangé à l'extérieur de la chambre de traitement (1) et connecté à un ordinateur, détecte avant l'entrée le début (17b) et la fin (17c) d'un récipient (17) ou d'un emballage se trouvant sur la bande transporteuse en circulation, en ce qu'un capteur de déplacement (26) est prévu qui sert à détecter la vitesse de la bande transporteuse et qui est également connecté à l'ordinateur (12), en ce qu'un générateur micro-ondes (8, 9), qui peut être réglé individuellement en fonction de sa puissance, est arrangé pour chaque canal d'alimentation (6, 7) et en ce que les générateurs micro-ondes (8, 9) sont connectés à l'ordinateur qui les met en marche et les arrête et qui règle leur puissance émise.

4. Appareil selon revendication 3,
   **caractérisé**
   en ce que le dispositif (15) repérant le début (17b) et la fin (17c) des récipients (17) ou des emballages avant ou lors de l'entrée dans la chambre de traitement (1) est conçu comme barrage photoélectrique à réflexion travaillant en commun avec le capteur de déplacement (26) et transmettant la position des récipients (17) ou des emballages sur la bande transporteuse (2) à l'ordinateur (12) pour le procédé de branchement et de coupure des générateurs individuels (8, 9).

5. Appareil selon revendication 3,
   **caractérisé**
   en ce que le dispositif (15), qui répère le début (17b) et la fin (17c) des récipients (17) ou des emballages sur la bande transporteuse (2) et qui est connecté à l'ordinateur, est conçu comme bouton-poussoir mécanique, bouton-poussoir optique, bouton-poussoir à laser, filière à air sous pression dynamique, détecteur de proximité ou comme bouton-poussoir à ultrasons ou à infrarouge.

6. Appareil selon revendication 3
   **caractérisé**
   en ce qu'un canal d'alimentation (6) pénétrant verticalement par le haut dans la chambre de traitement (1) est arrangé en alternance - vue en direction de travail de l'installation (1) l'un derrière l'autre - avec un canal d'alimentation (7) pénétrant verticalement par le bas dans la chambre (1), ou
   en ce qu'à l'opposé de chaque canal d'alimentation vertical supérieur (6) est arrangé un canal d'alimentation vertical inférieur (7).

Fig.1

EP 0 358 876 B1

Fig.2

Fig.3

# F i g.4

(Stand der Technik)

| | | |
|-----|-----|-----|
| 83° | 74° | 73° |
| 84° | 65° | 81° |
| 84° | 73° | 78° |

# F i g.5

| | | |
|-----|-----|-----|
| 63° | 64° | 63° |
| 64° | 65° | 64° |
| 64° | 63° | 63° |

# F i g.6